# FASCICULE DE BREVET EUROPEEN

(11) **EP 0 623 167 B1**
(45) Date de publication et mention de la délivrance du brevet: **15.12.1999**
(21) Numéro de dépôt: 93914480.4
(22) Date de dépôt: 13.01.1993
(51) Int. Cl.: C12N 7/02, C12N 7/06, C12N 7/08, A61K 39/12, G01N 33/569

(54) **PREPARATION D'ANTIGENES ET DE VACCINS DE VIRUS DE LA MYSTERY DISEASE, ANTIGENES ET VACCINS OBTENUS POUR LA PREVENTION DE CETTE MALADIE**
HERSTELLUNG VON ANTIGENEN UND IMPFSTOFFEN VOM MYSTERIE-DISEASE-VIRUS, ANTIGENE UND ERWORBENE IMPFSTOFFE ZUR VERHINDERUNG DIESER KRANKHEIT
PREPARATION OF MYSTERY DISEASE VIRUS ANTIGENS AND VACCINES AND ANTIGENS AND VACCINES PRODUCED FOR THE PREVENTION OF THIS DISEASE

(30) Priorité: 14.01.1992 FR 9200294
(43) Date de publication de la demande: 09.11.1994
(73) Titulaire: MERIAL, 69002 Lyon (FR)
(72) Inventeur: BRUN, André, F-69300 Caluire (FR); TARDY, Marie-Claude, F-69003 Lyon (FR); VAGANAY, Alain, F-69100 Villeurbanne (FR); VANDEPUTTE, Joris, F-38790 Diemoz (FR)
(74) Mandataire: Bernasconi, Jean
(86) Numéro de dépôt international: FR9300026
(87) Numéro de publication internationale: WO9314196

(56) Documents cités:
- EP-A- 0 541 418
- PHIND Database, PJB Publications Ltd, (Surrey, GB), abrégé no. 00278268, "Dutch team isolates mystery pig disease agent", & ANIMAL-PHARM 230, 21 JUNI 1991, P21
- World Patents Index Latest, semaine 8741, AN=87-286929, Derwent Publications Ltd, (Londres, GB), & JP,A,62198626 (ZH BISEIBUTSU KAGAKU KEN) 2 septembre 1987, voir abrégé
- World Patents Index Latest, semaine 8113, AN=81-215210, Derwent Publications Ltd, (Londres, GB), & DD,A,145705 (SOLISCH P) 7 janvier 1981, voir abrégé
- World Patents Index Latest, semaine 8408, AN=84-044721, Derwent Publications Ltd, (Londres, GB), & JP,A,50052224 (SHIKENJOCHO K E) 9 mai 1975, voir abrégé
- World Patents Index Latest, semaine 9142, AN=91-308267, Derwent Publications Ltd, (Londres, GB), & SU,A,1604851 (UKR AGRIC. MICROBIOL.) 7 novembre 1990, voir abrégé
- World Patents Index Latest, semaine 8432, AN=84-201354, Derwent Publications Ltd, (Londres, GB), & WO-A-8402847 (AMGEN) 2 août 1984, voir abrégé
- World Patents Index Latest, semaine 8325, AN=83-59978K, Derwent Publications Ltd, (Londres, GB), & JP,A,58079929 (ZH BISEIBUTSU KAGAKU KEN) 13 mai 1983, voir abrégé
- PHIND Database, PJB Publications Ltd, (Surrey, GB), abrégé no. 00275332, "Bonn symposium seeks fresh clues to mystery pig disease", & ANIMAL-PHARM 228, 17 MAI 1991, P3
- Medline Database, (Bathesda, US), abrégé no. 92303912, E. ALBINE et al.: "An enzyme linked", & ANN. RECH. VET., 1992, 23(2), P167-76
- Medline Database, (Bathesda, US), abrégé no. 92303911, T. BARON et al.: "Report on the first outbreaks", & ANN. RECH. VET., 1992, 23(2), P161-6
- Medline Database, (Bathesda, US), abrégé no. 92314095, D.A. BENFIELD et al.: "Characterization of swine", & J. VET. DIAGN. INVESt., 1992, 4(2), p127-33

## Description

La présente invention a trait à la préparation d'antigènes et de vaccins de virus de la Mistery Disease, ainsi qu'aux antigènes et aux vaccins obtenus.

La maladie dite Mystery Disease (M.D.) ou maladie mystérieuse ou encore Porcine Reproductive Respiratory Syndrome (P.R.R.S.), a commencé à être individualisée, chez le porc, en 1986 aux Etats-Unis et en 1990 en Europe. Cette maladie se traduit essentiellement chez le porc, par des signes d'abattement, une anorexie, et une hyperthermie de l'ordre de 40°C, que l'on observe classiquement sur les truies dans les élevages atteints par la maladie. Ces signes sont accompagnés ou suivis de troubles de la reproduction (mises bas précoces ou tardives et naissance de porcelets mort-nés, momifiés ou chétifs et retours en chaleurs des truies). Un syndrome respiratoire peut être observé chez les porcelets avec des lésions de pneumonie interstitielle. Des porcs plus âgés peuvent également être atteints de troubles respiratoires. Toute cette symptomatologie peut être accompagnée de maladies provoquées par des infections occasionnelles classiquement observées chez le porc.

Dans G. Wensvoort et al., Mystery Swine Disease in the Netherlands : the isolation of Lelystad virus, The Veterinary Quarterly, Vol. 13, N° 3, 19 juillet 1991, on décrit l'isolation d'un agent associé à la maladie dite Mystery Disease, qui est caractérisé comme un virus, désigné Lelystad virus, et qui est présenté comme l'agent causal de la maladie. Cette découverte pouvait constituer un premier pas pour la recherche d'un vaccin contre cette maladie.

Un procédé de production industrielle de ce virus ou d'antigène de ce virus n'était pas disponible, la culture ne paraissant possible que sur les macrophages alvéolaires du porc.

Or, les inventeurs ont réussi à isoler et à identifier un autre virus, responsable de cette maladie. Ce virus est de type Myxovirus, selon l'analyse réalisée au microscope électronique, et a comme caractéristique de ne pas être neutralisé par des sérums antigrippe porcine H1N1 et H3N2.

Une souche de ce virus identifiée sous la désignation P129-294 a été déposée à la Collection Nationale de Cultures de Micro-organismes tenue à l'Institut Pasteur sous le n° I-1153.

Dans la demande de brevet français n° 91 13338 déposée le 29 octobre 1991, les inventeurs ont décrit un procédé d'isolement du virus et son utilisation pour la préparation d'antigènes, un procédé de production industrielle de ce virus, ainsi que les vaccins réalisés à partir des antigènes précités contenant une quantité vaccinante efficace d'antigènes, dans des véhicules convenables.

Or, parallèlement à la découverte et à l'utilisation de cet autre virus, dont une souche a été déposée sous la désignation P129-294, les inventeurs ont isolé, à partir du même organe de la même truie provenant d'un élevage de porc d'Allemangne et identifiée sous le n° 294, un virus tout à fait différent, dont une souche identifiée sous la désignation P129-294B. Ils ont également isolé une autre souche virale à partir d'organes d'un porcelet d'un élevage d'Allemagne, prélevés le 13 février 1991, cette souche étant identifiée sous la désignation P120-117B.

Les inventeurs ont pu établir que les deux virus, à savoir le virus de type Myxovirus (par exemple P129-294), ci-après désigné virus A, et les virus correspondants aux souches P129-294B ou P120-117B, ci-après appelé virus B, se retrouvent associés dans les prélèvements effectués à partir de porcs malades ou infectés et dans les mêmes organes, et peuvent être considérés comme agents conjoints responsables de la maladie dite Mystery Disease.

L'invention a pour objet un procédé d'isolement de mélange de virus (A, B) de la Mystery Disease, à savoir un myxovirus (A) du type correspondant à la souche P129-294 déposée à la CNCM sous le n° I-1153 et un virus (B) correspondant à la souche P120-117B déposée à la CNCM sous le n° I-1163, comportant le prélèvement d'organes ou de sang de porcs malades ou infectés et des passages du surnageant de broyage ou de constituants du sang sur des cellules sensibles hétérologues ou homologues, puis la récolte du surnageant.

Les cellules sensibles homologues, peuvent être notamment des cellules primaires de porc, des cellules de lignée porcine ou hétérologue, telles que les cellules de la lignée Vero, MDCK (en présence de trypsine), ST, BHK.

On préfère effectuer le prélèvement à partir de poumon d'animal infecté ou encore d'un pool d'organes comprenant, par exemple, coeur, rate, foie, rein et tissus lymphoïdes.

Les récoltes virales n'hémagglutinent pas les globules rouges de poule, sont sensibles au chloroforme, réagissent avec des sérums convalescents provenant d'élevages infectés comme il a été démontré par immunofluorescence indirecte. La structure en microscopie électronique démontre des structures enveloppées d'une grandeur d'environ 50 nm.

L'invention a également pour objet un procédé de production industrielle de mélange de virus (A, B) de la Mystery Disease, à savoir un myxovirus (A) du type correspondant à la souche P 129-294 déposée à la CNCM sous le n° I-1153 et un virus (B) correspondant à la souche P 120-117 B déposée à la CNCM sous le n° 1-1163, dans lequel on cultive le virus sur des cellules sensibles homologues, des cellules primaires de porcs, des cellules de lignée porcine, ou hétérologues, notamment des cellules de la lignée Vero, MDCK, ST, BHK, et l'on récolte le dernier surnageant.

Le virus peut être utilisé pour la préparation d'antigènes.

Le procédé de production industrielle du virus A, décrit dans la demande de brevet français précitée avait, de façon remarquable, produit simultanément le virus B.

Le virus, ou le mélange de virus, récolté peut être utilisé pour la préparation d'antigènes. A cette fin, il est, de préférence, convenablement purifié selon des procédures usuelles, par exemple l'ultra-centrifugation ou la chromatographie. Il peut également être concentré par les techniques usuelles.

Selon l'utilisation envisagée, les préparations d'antigènes selon l'invention peuvent être constituées de particules virales vivantes, atténuées ou non, de particules inactivées, d'antigènes de sous-unités ou encore d'antigènes obtenus par recombinaison génétique à partir de gènes du ou des virus isolés, insérés pour être exprimés dans le génome d'hôtes recombinants procaryotes ou eucaryotes.

L'invention a également pour objet les vaccins réalisés à partir d'au moins un des antigènes précités, contenant, dans des véhicules convenables une quantité vaccinante d'une préparation d'antigène obtenue à partir de la souche virale P 120-117 B déposée à la CNCM sous le n° I-1163.

De préférence, les vaccins selon l'invention comportent les antigènes du virus A, en association avec des antigènes de virus B, et éventuellement d'autres antigènes infectieux pour l'immunisation du porc ou d'autres espèces animales.

Ces vaccins peuvent être prévus pour être administrés aux porcs mais également à d'autres animaux qui pourraient s'avérer sensibles à ce virus.

Un vaccin atténué peut être préparé par passages du virus sur culture cellulaire.

La quantité de chaque virus par dose vaccinale est de préférence comprise entre 10³ et 10⁸ DICC₅₀ par dose.

Le vaccin atténué vivant peut être présenté sous forme liquide ou lyophilisée en présence de stabilisateurs de formulations très variées, pouvant inclure des sucres, des protéines et des tampons. Le vaccin peut être adjuvé à l'aide d'adjuvants minéraux ou organiques.

Le vaccin vivant peut être administré à des animaux pour les protéger dès le début de la période d'engraissement ou avant l'insémination artificielle ou la saillie, ou au cours de la gestation, en une et de préférence en deux injections à trois ou quatre semaines d'intervalle.

Un vaccin inactivé peut être préparé à partir de suspensions virales obtenues par passages sur des systèmes cellulaires homologues (porcin) ou hétérologues, puis inactivation par des agents inactivants chimiques usuels tels que la Béta-propiolactone, des enzymes ou des solvants organiques ou des détergents. L'inactivation peut également être obtenue par action physique tels que rayonnement ultra-violet, irradiations gamma ou rayons X. L'agent inactivant peut être neutralisé si nécessaire.

Le vaccin inactivé contient de préférence au moins l'équivalent de 10⁵ DICC₅₀ de chaque virus par dose vaccinale, concentration déterminée avant inactivation.

Le vaccin inactivé peut être administré aux animaux pour les protéger dès le début de la période d'engraissement, ou avant l'insémination artificielle ou la saillie, ou en cours de gestation, en une et de préférence deux injections à trois ou quatre semaines d'intervalle. on préfère que le vaccin contienne un adjuvant d'origine minérale ou organique.

Un vaccin recombinant peut être obtenu, par exemple, par insertion de la séquence codant pour l'antigène recherché du virus dans le génome de l'hôte. L'hôte peut être un virus, notamment pour la réalisation d'un vaccin vivant.

Cet hôte peut également être un système bactérien, de levure, ou d'autres cellules eucaryotes. Dans ce cas, l'hôte est de préférence utilisé pour la production d'antigènes qui sont ensuite purifiés et conditionnés pour faire le vaccin.

Le vaccin peut être présenté sous forme monovalente ou associé à d'autres agents viraux ou bactériens responsables de maladies chez le porc.

L'invention a également pour objet des préparations d'antigènes à fin de diagnostic, caractérisées en ce qu'elles comportent des antigènes de virus A ou un mélange d'antigènes de virus A et B. Ces préparations d'antigènes à fin de diagnostic sont réalisées de façon usuelle et comprennent les moyens usuels permettant l'identification et éventuellement la quantification des réactions sérologiques positives.

L'invention a également pour objet les préparations d'anticorps contre ces antigènes, utilisables pour le diagnostic de la maladie.

D'autres avantages et caractéristiques de l'invention apparaîtront à la lecture de la description suivante, faite à titre d'exemple non limitatif.

### Exemple 1 : Isolement du virus P129-294B.

Des prélèvements de poumon ont été réalisés sur une truie provenant d'un élevage de porcs d'Allemagne et identifiée sous le n° 294.
**a)** Méthode :
   Broyats d'organes : chaque prélèvement est broyé individuellement dans milieu MEM + antibiotiques.
   Dilution : poids organe par volume d'environ 1 pour 10.
   Centrifugation clarifiante pour récupération du liquide surnageant (LS).
   Filtration à 0,22 µ.
**b)** Essais sur cultures cellulaires :
   Premiers passages : le LS est inoculé sur couche cellulaire établie de macrophages de poumon de porc SPF. Trois jours après inoculation, il a été observé un effet cytopathique se manifestant par une modification morphologique des macrophages, à différents stades d'évolution. Cet effet cytopathique est maximal le cinquième jour.
   Examen en microscopie électronique : méthode sur coupes de macrophages infectés et sains. Les macrophages infectés présentent un aspect totalement différent des macrophages sains. On note à l'intérieur des cellules infectées des vacuoles contenant des particules d'environ 50 nm.

### Exemple 2 : Isolement du virus P120-117B des organes d'un porcelet prélevé le 13 février 1991.

**a)** Méthode :
   Broyats d'organes : l'organe est broyé dans du milieu MEM plus antibiotiques.
   Dilution : poids de l'organe par volume d'environ 1 pour 10.
   Centrifugation clarifiante pour récupération du liquide surnageant (LS).
   Filtration à 0,22 µ.
**b)** Essais sur cultures cellulaires :
   Premiers passages : le LS est inoculé sur couche cellulaire établie de macrophages de poumon de porc SPF. Trois jours après inoculation, il a été observé un effet cytopathique se manifestant par une modification morphologique des macrophages, à différents stades d'évolution. Cet effet cytopathique est maximal le cinquième jour.

### Exemple 3 : Etude sérologique.

Les antigènes constitués par les préparations virales P129/294 B et P120/177 B, ainsi que par deux autres souches virales P 206 et P 208 ont été testés vis-à-vis :
- d'un sérum de référence usuel, dit de Ploufragan ;
- de sérums de truies ayant servi à la reproduction expérimentale de la maladie (prélèvement 21 jours après inoculation et après la mise-bas de truies) ;
- des sérums de porcelets inoculés expérimentalement par voie intranasale.

Ce bilan sérologique par réaction d'immunofluorescence indirecte est le suivant :

| | P129/294 B | P120/117 B | P206 ou P 208 |
|---|---|---|---|
| Sérums Ploufragan | + | + | + |
| | | | |
| Sérums truies | + | - | - |
| | | | |
| Sérums porcelets | - | + | + |

### Exemple 4 : Etude du pouvoir pathogène comparé des deux virus : P129-294 et P129-294B.

**a)** La souche virale P129-294 : l'agent viral isolé sur oeufs a été inoculé sous forme d'une suspension virale de liquide allantoïdien du troisième passage, à 2 porcs SPF, par voie intraveineuse, sous un volume de 1 cm³. 2 autres porcs SPF ont été inoculés également par voie intraveineuse avec du virus Newcastle, souche Texas. 2 porcs ont été conservés comme témoins et inoculés avec du liquide allantoïdien non dilué. Les animaux étaient stabulés dans des boxes différents. Ils ont été observés durant 14 jours avec prise de température quotidienne et pesée aux jours 0 et 7. Des prélèvements de sang ont été réalisés aux jours 0 et 14.
   Les 2 porcs inoculés avec l'agent hémagglutinant ont présenté une hyperthermie comprise entre 39°5 et 40°C entre le jour 1 après inoculation et le jour 7, alors que les 4 autres porcs n'ont jamais présenté cette élévation de température. Il a été noté, au jour 2, des traces de vomissement dans le box des 2 porcs inoculés avec l'agent hémagglutinant. D'autre part, une inappétence de quelques jours a été observée chez ces 2 animaux, ce qui s'est traduit par une évolution pondérale très nettement défavorable pour ces 2 animaux par rapport à ceux inoculés soit avec liquide allantoïdien témoin, soit avec du virus de Newcastle. Le graphique ci-après illustre cette différence de croissance. A l'autopsie, l'un des 2 porcs inoculés avec l'agent hémagglutinant présentait des lésions peu marquées de pneumonie.
**b)** La souche virale P129-294B a été inoculée à 2 truies, par voie nasale, avec 10^{3,5} doses infectieuses de l'isolat aux 58ème et 66ème jours de la gestation. Les truies n'ont présenté aucun signe clinique de maladie après inoculation. Au jour 110 de la gestation, la truie n°25 a mis bas de 2 porcelets morts-nés et de 8 porcelets vivants. Les vivants avaient un aspect chétif et étaient incapables de marcher. Leur comportement ressemblait exactement à celui observé chez des porcelets dans les élevages infectés. 3 porcelets sont morts le jour de la mise bas, 2 à 24 heures, 3 à 72 heures et, enfin, 1 porcelet a été trouvé mort à 72 heures après mise bas tardive. A ce jour, la deuxième truie, en gestation plus précoce, à présenté fièvre et anorexie au jour 106 de gestation. A 112 jours de gestation, elle a mis bas 5 porcelets morts-nés et un porcelet vivant très chétif.

**Conclusion :** Le virus P129-294 est responsable de signes cliniques sur des porcelets, apparaissant sous forme d'hyperthermie et d'anorexie, alors que le virus P129-294B est responsable de troubles de la reproduction. Cet ensemble clinique reproduit les observations faites dans les élevages infectés. Enfin, ces deux virus ont été isolés du même organe, le poumon, d'une même truie.

### Exemple 5 : Etude du pouvoir pathogène du virus 120/117 B.

Une inoculation expérimentale réalisée sur des porcelets d'un poids de 20 kg environ, à l'aide de la souche P120/117 B, administrée par voie intranasale avec un inoculum titrant 10^{5,5} DICC₅₀/ml entraîne une fièvre et une inappétence pendant quelques jours.

### Exemple 6 : Suivi sérologique. Présence d'anticorps contre la souche P129-294.

238 sérums provenant d'élevages infectés ont été examinés pour la présence d'anticorps vis-à-vis de la souche P129-294.

Dans ces élevages, des signes manifestes de maladie mystérieuse du porc ont été observés (avortements, problèmes respiratoires). La méthode sérologique est un test Elisa indirect ; l'antigène utilisé est un antigène obtenu sur oeufs après ultracentrifugation. Les sérums à tester sont traités au prélable à l'aide d'un antigène oeuf négatif ayant subi le même traitement d'ultracentrifugation. Les 238 sérums provenant d'élevages infectés d'Allemagne, de Belgique ou de Hollande, présentent des anticorps très positifs pour 43 % d'entre eux, faiblement positifs pour 37 % et négatifs pour 20 %.

**Conclusion :** Dans les cas cliniques de maladie mystérieuse, on observe une sérologie positive contre la souche P129-294 dans 80 % des cas. Il faut remarquer que des sérums provenant d'élevages non suspects, sont négatifs, ou que la fréquence de positivité est significativement inférieure a celle observée dans les élevages cliniquement atteints.

Ces résultats montrent l'importance de l'infection à Myxovirus dans le syndrome maladie mystérieuse.

### Exemple 7 : Préparation de vaccin vivant.

Les virus P129-294 et P120-117B ou P129-294B sont multipliés par passages sur un ou des systèmes cellules adaptés. La récolte est traitée suivant le vaccin qui est produit : vaccin lyophilisé pour reprise en solvant aqueux, en solvant huileux ou en d'autres vaccins. Il peut aussi être présenté en vaccin liquide en solvant aqueux, huileux ou autre vaccin.

### Exemple 8 : Préparation de vaccin inactivé.

Les virus P129-294 et P120-117B ou P129-294B sont multipliés sur cellules BHK, Vero, macrophages, ou cellules de lignée porcine, et inactivés par la Béta-propiolactone, éthylène-imine, puis adjuvés avec un adjuvant aqueux ou huileux.

Les souches virales P120-117B et P129-294B ont été déposées auprès de la Collection Nationale de Cultures de Microorganismes (CNCM) :
P120-117B déposée sous le n° I-1163
P129-294B déposée sous le n° I-1164.
Le virus P120-117B apparaît comme un togavirus.

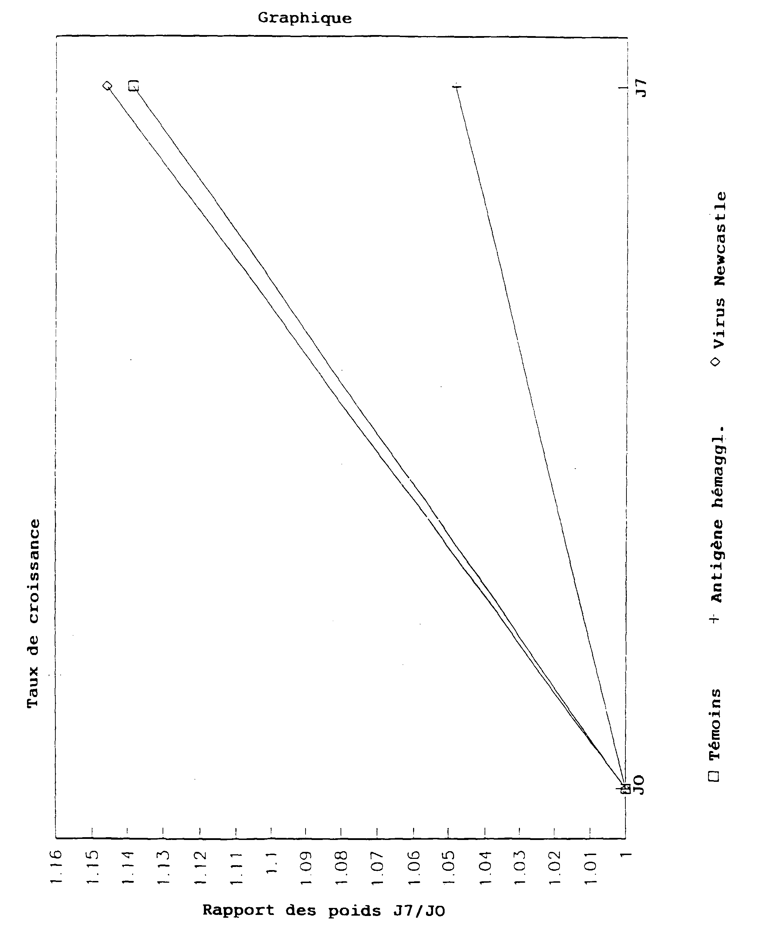

## Revendications

1. Procédé d'isolement de mélange de virus (A, B) de la Mystery Disease, à savoir un myxovirus (A) du type correspondant à la souche P129-294 déposée à la CNCM sous le n° 1-1153 et un virus (B) correspondant à la souche P120-117B déposée à la CNCM sous le n° 1-1163, comportant le prélèvement d'organes ou de sang de porcs malades ou infectés et des passages du surnageant de broyage ou de constituants du sang sur des cellules sensibles hétérologues ou homologues, puis la récolte du surnageant.

2. Procédé selon la revendication 1, caractérisé en ce que les passages sont effectués sur cellules de la lignée Vero, MDCK, ST, BHK.

3. Procédé de production industrielle de mélange de virus (A, B) de la Mystery Disease, à savoir un myxovirus (A) du type correspondant à la souche P 129-294 déposée à la CNCM sous le n° 1-1153 et un virus (B) correspondant à la souche P 120-117 B déposée à la CNCM sous le n° 1-1163, dans lequel on cultive le virus sur des cellules sensibles homologues, des cellules primaires de porcs, des cellules de lignée porcine, ou hétérologues, notamment des cellules de la lignée Vero, MDCK, ST, BHK, et l'on récolte le dernier surnageant.

4. Préparation purifiée de la souche virale P 120-117 B déposée à la CNCM sous le n° 1-1163.

5. Préparation antigénique comprenant des particules vivantes, atténuées ou non, ou des particules inactivées, ou des antigènes de sous-unité virale, obtenus à partir de la préparation selon la revendication 4.

6. Vaccin contre la Mystery Disease caractérisé en ce qu'il contient, dans un véhicule approprié, une quantité vaccinante d'une préparation selon l'une des revendications 4 et 5.

7. Vaccin atténué selon la revendication 6, caractérisé en ce qu'il est préparé par passage du ou des virus concernés sur culture cellulaire.

8. Vaccin atténué selon la revendication 7, caractérisé en ce que la quantité de virus par dose vaccinale est comprise entre 10³ et 10⁸ DICC₅₀.

9. Vaccin inactivé selon la revendication 6, caractérisé en ce que le surnageant de culture est inactivé par un agent inactivant chimique ou physique.

10. Vaccin inactivé selon la revendication 9, caractérisé en ce qu'il contient au moins l'équivalent de 10⁵ DICC₅₀ de virus par dose vaccinale.

11. Préparation à usage de diagnostic de la maladie dite de la Mystery Disease, caractérisée en ce qu'elle comporte une préparation selon l'une des revendications 4 et 5 ou des anticorps contre une telle préparation.

## Claims

1. Method for isolation of a mixture of viruses (A, B) of the Mystery Disease, i.e. a myxovirus (A) of the type corresponding to the strain P129-294 filed at the CNCM under the number 1-1153 and a virus (B) corresponding to the strain P120-117B filed at the CNCM under the number I-1163, comprising the removal of organs or of blood of sick or infected pigs, and treatments of the floating matter from pulvensation or from the constituents of the blood with sensitive heterologous or homologous cells, thereafter the extraction of the floating matter.

2. Method according to claim 1, characterised in that the treatments are effected upon cells of the line Vero, MDCK, ST, BHK.

3. Method for industrial production of a mixture of viruses (A, B) of the Mystery Disease, i.e. a myxovirus (A) of the type corresponding to the strain P129-294 filed at the CNCM under the number 1-1153 and a virus (B) corresponding to the strain P 120-117B filed at the CNCM under the number 1-1163, in which the virus is cultivated on sensitive homologous cells, primary cells from pigs, cells from the pig line, or heterologous cells, particularly cells of the line Vero, MDCK, ST, BHK, and the final floating matter is extracted.

4. Purified preparation of the viral strain P 120-117 B filed at the CNCM under the number 1-1163.

5. Antigenic preparation comprising living particles, attenuated or not, or inactivated particles, or viral sub-unit antigens, obtained from the preparation according to claim 4.

6. Vaccine against the Mystery Disease characterised in that it contains, in an appropriate vehicle, a vaccinating quantity of a preparation according to one of the claims 4 and 5.

7. Attenuated vaccine according to claim 6, characterised in that it is prepared by the treatment of the virus or viruses concerned with the cellular culture.

8. Attenuated vaccine according to claim 7, characterised in that the quantity of virus per vaccinal dose lies between 10³ and 10⁸ DICC₅₀.

9. Inactivated vaccine according to claim 6, characterised in that the floating matter of the culture is inactivated by a chemical or physical inactivating agent.

10. Inactivated vaccine according to claim 9, characterised in that it contains at least the equivalent of 10⁵ DICC₅₀ of virus per vaccinal dose.

11. Preparation for the diagnosis of the disease described as Mystery Disease, characterised in that it comprises a preparation according to one of the claims 4 and 5 or antibodies against such a preparation.

## Patentansprüche

1. Verfahren zur Isolierung der Mischung von Viren (A, B) der Mystery Disease, das heißt eines Myxovirus (A) des Typs, der dem Stamm P129-294 entspricht, der beim CNCM unter der Nr. I-1153 hinterlegt worden ist, und eines Virus (B), der dem Stamm P120-117B entspricht, der beim CNCM unter der Nr. I-1163 hinterlegt worden ist, umfassend die Entnahme von Organen oder Blut von kranken oder infizierten Schweinen und Überleiten des Vermahlungsüberstands oder von Blutbestandteilen über heterologe oder homologe sensible Zellen und Gewinnung des Überstands.

2. Verfahren nach Anspruch 1, dadurch gekennzeichnet, daß die Überleitungen über Zellen der Zellinie Vero, MDCK, ST, BHK bewirkt werden.

3. Verfahren zur technischen Herstellung der Mischung von Viren (A, B) der Mystery Disease, das heißt eines Myxovirus (A) des Typs, der dem Stamm P 129-294 entspricht, der beim CNCM unter der Nr. I-1 153 hinterlegt worden ist, und eines Virus (B), der dem Stamm P 120-117 B entspricht, der beim CNCM unter der Nr. I-1163 hinterlegt worden ist, gemäß dem das Virus auf homologen sensiblen zellen, primären Schweinezellen, Zellen der Schweine-Zellinie oder heterologen Zellen, insbesondere zellen der Linie Vero, MDCK, ST, BHK gezüchtet wird und der letzte Überstand gewonnen wird.

4. Gereinigtes Präparat des Virenstammes P 120-1 17 B, der unter der Nr. I-1163 beim CNCM hinterlegt worden ist.

5. Antigenes Präparat umfassend gegebenenfalls geschwächte lebende Teilchen oder inaktivierte Teilchen oder Antigene für eine virale Untereinheit, erhalten ausgehend von dem Präparat nach Anspruch 4.

6. Impfstoff gegen die Mystery Disease, dadurch gekennzeichnet, daß er in einem geeigneten Träger eine impfende Menge eines Präparats nach einem der Ansprüche 4 und 5 enthält.

7. Abgeschwächter Impfstoff nach Anspruch 6, dadurch gekennzeichnet, daß er durch Überleiten des oder der betreffenden Viren über eine Zellkultur hergestellt worden ist.

8. Abgeschwächter Impfstoff nach Anspruch 7, dadurch gekennzeichnet, daß die Virenmenge pro Impfdosis zwischen 10³ und 10⁸ DICC₅₀ liegt.

9. Inaktivierter Impfstoff nach Anspruch 6, dadurch gekennzeichnet, daß der Überstand der Kultur mit einem chemischen oder physikalischen inaktivierenden Mittel inaktiviert worden ist.

10. Inaktivierter Impfstoff nach Anspruch 9, dadurch gekennzeichnet, daß er mindestens das Äquivalent von 10⁵ DICC₅₀ des Virus pro Impfdosis enthält.

11. Präparat zur Verwendung als Diagnostikum für die als Mystery Disease bezeichnete Erkrankung, dadurch gekennzeichnet, daß es ein Präparat nach einem der Ansprüche 4 und 5 und Antikörper gegen ein solches Präparat umfaßt.
